# EUROPEAN PATENT SPECIFICATION

(11) **EP 4 049 709 B1**
(45) Date of publication and mention of the grant of the patent: **09.04.2025**
(21) Application number: 20879880.1
(22) Date of filing: 23.10.2020
(51) Int. Cl.: A61M 39/28, A61M 5/168, F16B 2/22, F16L 33/02, F16B 2/10, F16L 55/10

(54) **MEDICAL TUBE CLAMP**
MEDIZINISCHE ROHRKLEMME
PINCE À TUBE MÉDICAL

(30) Priority: 25.10.2019 JP 2019194009
(43) Date of publication of application: 31.08.2022
(73) Proprietor: Nipro Corporation, Osaka-shi, Osaka 531-8510 (JP)
(72) Inventor: YAMAGUCHI, Takeshi, Osaka-shi, Osaka 531-8510 (JP)
(74) Representative: Balder IP Law, S.L.
(86) International application number: PCT/JP2020/039878
(87) International publication number: WO 2021/079980

(56) References cited:
- EP-A1- 0 637 456
- WO-A1-2012/111310
- WO-A1-2015/064233
- JP-A- 2001 527 441
- US-A1- 2013 310 768

## Description

### TECHNICAL FIELD

The present invention relates to a medical tube clamp, and in particular to a clamp that presses and occludes a flexible medical tube. The present invention is defined by the appended claims.

### BACKGROUND

In the related art, in the medical field, a flexible medical tube is used as a tube element which is a part of an extracorporeal circulation circuit of a body fluid such as blood, an auxiliary circuit connected to the extracorporeal circulation circuit, an infusion line of medical fluid, or the like. In some cases, the medical tube is equipped with a clamp for pressing and occluding the tube at a desired position. As the clamp applied for the medical tube, a clamp which is made of a resin and which can be manipulated with one hand is known.

For example, Patent Document 1 discloses a clamp made of a resin, wherein a protrusion that presses a tube is formed on each of opposing surfaces of two plates which are connected to each other, and there is provided a locking unit that fixes the plates at positions at which the plates are closer to each other and that maintains an occluded state of the tube. The locking unit fixes (locks) the plates in a state in which the two plates are closer to each other and press the tube, to thereby maintain the occluded state of the tube. The clamp disclosed in Patent Document 1 has a centering function to guide the plates to predetermined normal positions so that the plates do not significantly deviate from the predetermined position at which a normal locked state is realized.

### CITATION LIST

### PATENT DOCUMENT

Patent Document 1: WO 2012/111310

### SUMMARY

### TECHNICAL PROBLEM

In some cases, the medical tube is not viewed while being manipulated, the clamp is locked, and the tube is not pressed normally. In this case, an occlusion property of the tube is reduced and leakage is caused. As a mechanism for preventing such a situation, the clamp of Patent Document 1 discloses a centering function for guiding the plates to normal positions. However, as a result of various reviews of the clamp of Patent Document 1, it was newly found that, in some cases, when the clamp is used for a tube of a large outer size, the clamp is locked while the centering function fails to be sufficiently realized. Specifically, a tube having a large outer size elastically deforms by the protrusions of the clamp at an early stage in the clamp manipulation. Because of this, a frictional force between each protrusion and the tube becomes significantly large due to a reaction force of the tube and an increase in a contact area in a width direction caused by widening of the tube in the width direction. Thus, a situation has been observed in which, even when the centering function acts, the plates do not move relative to each other in the width direction, due to the frictional force. In such cases, due to the structure of the centering function, the clamp should not be locked, because the plates which do not move relative to each other in the width direction also do not move in the direction toward each other. However, a situation was newly found in which, when an excessive pressurization force is applied to an end of the plate in the width direction, connecting portions deform while the plates are in a deviated state, the plates are thus inclined, a corner of the plate is locked to a locking portion, and the clamp is locked in a non-centered state.

### SOLUTION TO PROBLEM

According to the present invention, there is provided a medical tube clamp that presses and occludes a flexible medical tube, the medical tube clamp comprising: a first plate on which a first protrusion is formed; a second plate placed opposing the first plate, and on which a second protrusion that presses the medical tube along with the first protrusion is formed; a connecting portion that connects one end of the first plate in a length direction and one end of the second plate in a length direction, and that can elastically deform so as to cause the plates to be closer to each other; and a locking unit that is formed extending from the first plate toward the second plate, that locks the other end of the second plate in the length direction, and that maintains an occluded state of the medical tube, wherein a pair of sidewalls that extend in a standing manner are formed on respective ends in the width direction on the first plate, a sloped surface is formed on the second plate, the sloped surface being inclined toward the first plate from both ends in the width direction toward the center in the width direction of the second plate, and an inclination angle θ of the sloped surface with respect to the width direction of the second plate is 10° ~ 60°.

A medical tube clamp according to an aspect of the present invention desirably has a structure in which the second plate is inclined with respect to the first plate in an unlocked state so that a spacing between the two plates which press the tube is widened at the other end side in a length direction distanced from the connecting portion. In addition, the medical tube clamp according to an aspect of the present invention is desirable for a medical tube having a large outer size. For example, the medical tube clamp is desirable for a medical tube having an outer size which is equivalent to or larger than a spacing between the first protrusion and the second protrusion of the clamp before assembly; that is, a medical tube in which the first protrusion and the second protrusion of the clamp are in contact with an outer surface of the medical tube in the unlocked state. In the medical tube clamp according to an aspect of the present invention, the other end in the length direction of the second plate is pushed toward the first plate so that the spacing between the first protrusion and the second protrusion of the plates is reduced and the tube is pressed. The other end in the length direction of the second plate which is pushed is locked by the locking unit, to thereby maintain the locked state in which the tube is occluded.

With the medical tube clamp having the above-described structure, when an inappropriate manipulation is performed such as pushing the second plate in a slanted direction, the sloped surface of the second plate contacts the side wall of the first plate, and a contact point of the sloped surface with the side wall slides toward an upper end of the sloped surface. As a result, the second plate moves to an inner side of the clamp. That is, even when an inappropriate manipulation is performed, the second plate can be centered at the normal position at which the tube can be reliably occluded, and the normal locked state can be easily secured. Therefore, with the medical tube clamp having the above-described structure, the medical tube can be occluded quickly and reliably.

In the medical tube clamp according to an aspect of the present invention, the inclination angle θ of the sloped surface is 10° ~ 60°, and the sloped surface is a steeply inclined surface. Thus, a force in a lateral direction which acts from the inclined surface to the side wall is large. As a result, even in a case where the outer size of the medical tube is large or the like, the second plate is guided to the inner side against the frictional force, and, when an inappropriate manipulation is performed such as pushing the second plate in the slanted direction, the normal locked state can be easily secured. The inclination angle θ of the sloped surface is desirably 25° ~ 45°, and is more desirably 30° ~ 45°. According to this configuration, a force necessary for the centering of the second plate is significantly reduced, and smoother centering can be enabled. As a result, in a case where an inappropriate manipulation is performed such as pushing the second plate in the slanted direction, the normal locked state can even more easily secured.

Alternatively, a configuration may be employed in which one of the first protrusion and the second protrusion is more sharply pointed than the other of the first protrusion and the second protrusion. As a result, the force necessary for pressing the medical tube can be reduced, maneuverability can be improved, and an occlusion property of the medical tube can be improved. Because of this, a desirable clamp can be realized even for a medical tube having a small outer size for which occlusion is relatively difficult.

### ADVANTAGEOUS EFFECTS

According to an aspect of the present invention, a medical tube clamp can be provided in which the tube can be more reliably occluded even when an inappropriate manipulation is performed, while securing superior maneuverability. With the medical tube clamp of the present invention, for example, when a manipulation is performed such as pushing the second plate in a slanted direction, the second plate can be centered at the normal position at which the tube can be reliably occluded, and the normal locked state can be easily secured.

### BRIEF DESCRIPTION OF DRAWINGS

FIG. 1 is a perspective diagram of a medical tube clamp according to an embodiment of the present invention.
FIG. 2 is a side view of the medical tube clamp according to the embodiment.
FIG. 3 is a side view showing an unlocked state of the medical tube clamp according to the embodiment.
FIG. 4 is a side view showing a locked state of the medical tube clamp according to the embodiment.
FIG. 5 is a cross sectional diagram along a line AA in FIG. 2.
FIG. 6 is a cross sectional diagram along a line BB in FIG. 2.
FIG. 7 is a diagram showing a relationship between an inclination angle θ of a sloped surface and a force necessary for locking a clamp in the medical tube clamp according to the embodiment.
FIG. 8 is a diagram showing an alternative configuration of the sloped surface.
FIG. 9 is a diagram showing another alternative configuration of the sloped surface.

### DESCRIPTION OF EMBODIMENTS

An embodiment of the present invention will now be described in detail with reference to the drawings. The embodiment described below is merely exemplary, and the present invention is not limited to the embodiment. In the present invention, unless otherwise noted, a description of "a numerical value (A) ~ a numerical value (B)" means a value of greater than or equal to the numerical value (A) and less than or equal to the numerical value (B).

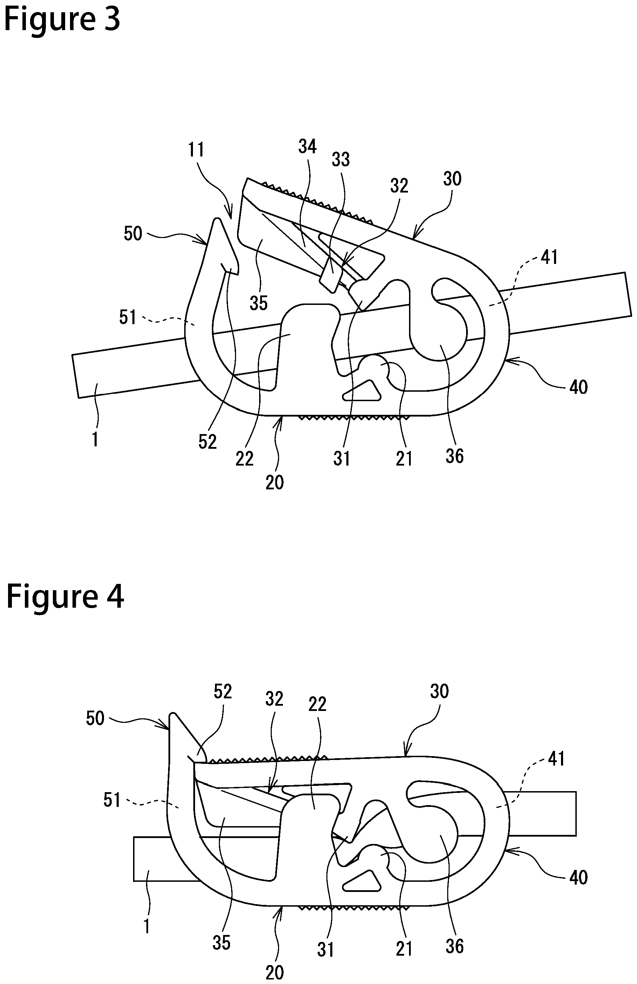

In the following, a case is exemplified in which a medical tube clamp 10 (hereinafter simply referred to as a "clamp 10") according to an embodiment of the present invention is equipped on a medical tube 1 exemplified in FIG. 3 or the like, but, alternatively, the clamp 10 may be applied to other medical tubes such as a tube having a smaller diameter than the medical tube 1. The medical tube on which the clamp 10 is equipped is a part of, for example, an extracorporeal circulation circuit, an auxiliary circuit, an infusion line, or the like.

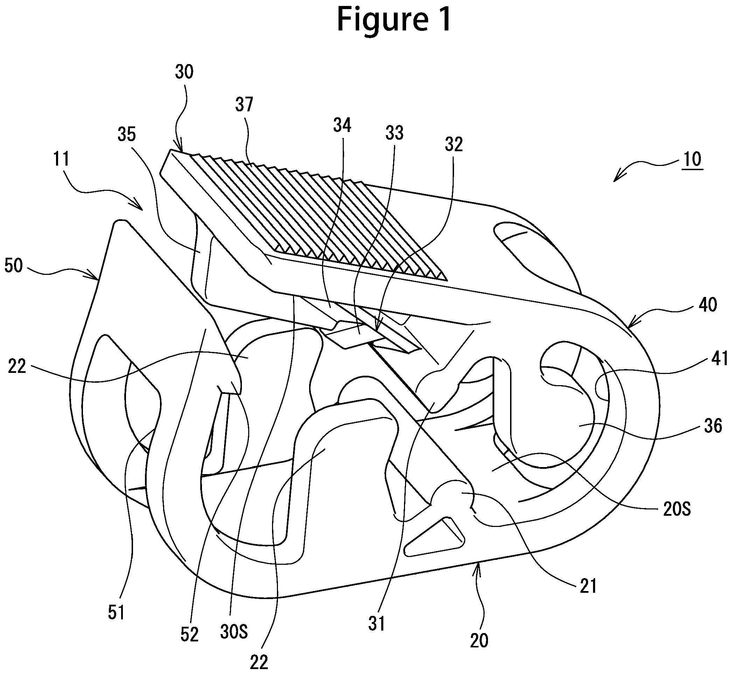

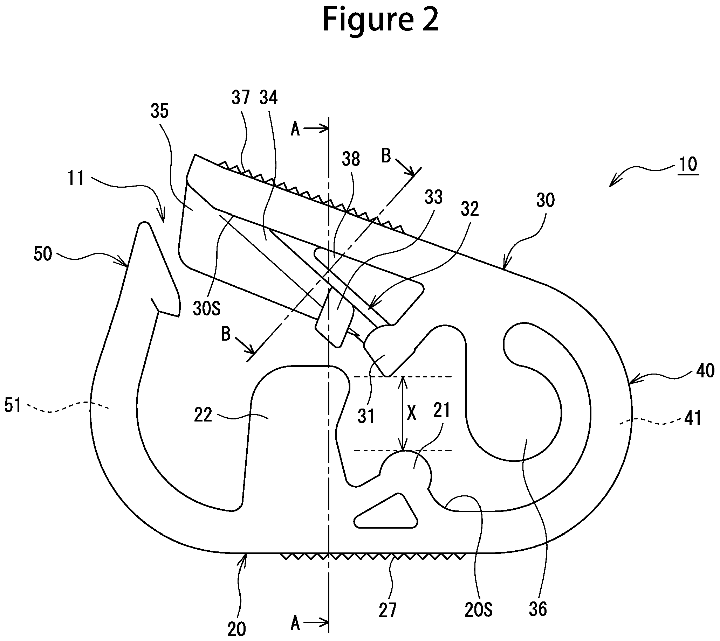

FIG. 1 is a perspective diagram of the clamp 10, and FIG. 2 is a side view of the clamp 10. FIGs. 3 and 4 are diagrams showing states in which the clamp 10 is equipped on the medical tube 1. FIG. 3 shows an unlocked state in which the medical tube 1 is not pressed or occluded, and FIG. 4 shows a locked state in which the medical tube 1 is pressed and occluded, and a second plate 30 is locked by a locking unit.

As shown in FIGs. 1 ~ 4, the clamp 10 is a molded structure made of a resin and having a first plate 20 on which a first protrusion 21 is formed, and the second plate 30 which is placed opposing the first plate 20 and on which is formed a second protrusion 31 which presses the medical tube 1 along with the first protrusion 21. The clamp 10 presses and occludes the medical tube 1 with the two plates. The second plate 30 is placed opposing the first plate 20 with a gap between the second plate 30 and the first plate 20 through which the medical tube 1 can be passed. Each of the first plate 20 and the second plate 30 has an approximate rectangular shape.

In the following, for the convenience of description, each of one end in a length direction of the first plate 20 and one end in a length direction of the second plate 30 connected by a connection portion 40 to be described below is referred to as a "base end", and each of the other ends in the length direction opposite from the base ends is referred to as a "tip end". The terms base end and tip end are similarly used for the clamp 10. Further, a direction along the length direction of each plate will be referred to as a "vertical direction" of the clamp 10, a direction along a width direction will be referred to as a "lateral direction", and a direction orthogonal to the vertical direction and the lateral direction will be referred to as an "up-and-down direction".

The clamp 10 is equipped at a desired position of the medical tube 1 in a state in which the length direction of each plate is along a length direction of the medical tube 1. The clamp 10 has a structure in which a spacing between the first plate 20 and the second plate 30 which press the medical tube 1 is widened at a tip end side of each plate, in an unlocked state in which the medical tube 1 is not occluded. In the clamp 10, a tip end portion of the second plate 30 is pressed toward the first plate 20 to reduce the spacing between the plates, so that a spacing X between the first protrusion 21 and the second protrusion 31 is reduced and the medical tube 1 is occluded. Here, the spacing X means a length along the up-and-down direction, from an upper end of the first protrusion 21 to a lower end of the second protrusion 31.

The clamp 10 has the connecting portion 40 which connects the tip end portions of the first plate 20 and the second plate 30. The connecting plate 40 is elastically deformable in the up-and-down direction so as to cause the first plate 20 and the second plate 30 to be closer to each other. In the clamp 10, with the elastic deformation of the connecting portion 40, when the second plate 30 is pressed or the pressing force is released, the spacing X between the first protrusion 21 and the second protrusion 31 changes, and the medical tube 1 is opened or closed.

The connecting portion 40 largely curves toward an outer side of the clamp 10, and is formed in an approximate U shape in side view. The spacing between the first plate 20 and the second plate 30 can be adjusted by changing a length in the up-and-down direction of the connecting portion 40 (diameter of an arc). A tube pass-through hole 41 is formed on the connecting portion 40 in a wide area other than respective end portions in the width direction. By forming the tube pass-through hole 41 large, it becomes possible to facilitate equipment of the clamp 10 on the medical tube 1, and to reduce rigidity of the connecting portion 40, which facilitates elastic deformation of the connecting portion 40.

In addition, the clamp 10 includes a locking portion 50 formed extending from the first plate 20 toward the second plate 30. The locking portion 50 is a locking unit which locks the tip end portion of the second plate 30 and maintains the occluded state of the medical tube 1. On a tip end portion of the locking portion 50, a hook 52 protruding to an inner side of the clamp 10 is formed, on which the tip end portion of the second plate 30 is hooked. In the present embodiment, a slanted surface inclined toward the inner side of the clamp 10 and in a slanted downward direction is formed at the tip end portion of the locking portion 50, and a lower end of the slanted surface protrudes to the inner side of the clamp 10 to form the hook 52. The slanted surface and the hook 52 are formed over the entire width of the locking portion 50.

The locking portion 50 is a plate-shaped portion extending from the tip of the first plate 20 in the upward direction, and is formed to have the same width as the first plate 20. In the present embodiment, the first plate 20, the second plate 30, the connecting portion 40, and the locking portion 50 are formed to have the same width, and, in the unlocked state and in the normal locked state, center parts of the two plates in the width direction overlap each other in the up-and-down direction. The locking portion 50 has a shape in which a base end portion close to the first plate 20 is curved toward the outer side of the clamp 10, and a tip end portion is slightly inclined toward the inner side. Moreover, a tube pass-through hole 51 is formed on the locking portion 50, similar to the connecting portion 40.

The locking portion 50 elastically deforms in the vertical direction of the clamp 10. When the tip end portion of the second plate 30 is pushed toward the first plate 20, the tip end portion contacts the slanted surface formed on the tip end portion of the locking portion 50, to thereby elastically deform the locking portion 50 toward the outer side, and the tip end portion moves over the hook 52 and close to the first plate 20. At this point, the locking portion 50 is restored to its original shape, and, when a hand is moved off from the second plate 30, the tip end portion of the second plate 30 is hooked and locked on the hook 52, and a locked state is realized in which the medical tube 1 is occluded.

On the other hand, when the tip end portion of the locking portion 50 is pushed to the outer side and is elastically deformed in the locked state, the second plate 30 is released from the hook 52 and the locked state is released. The second plate 30 returns to the position of the unlocked state due to a restoration force of the connecting portion 40. In the clamp 10 in the unlocked state, an opening 11 is formed between the tip end portion of the second plate 30 and the tip end portion of the locking portion 50. In the locked state, the opening 11 does not exist, and the first plate 20, the connecting portion 40, the second plate 30, and the locking portion 50 are connected in an annular shape.

The clamp 10 is equipped on the medical tube 1 by passing the medical tube 1 through the tube pass-through holes 41 and 51 of the connecting portion 40 and the locking portion 50, in a state in which the medical tube 1 is sandwiched between the first plate 20 and the second plate 30. In this process, the clamp 10 is equipped in such a manner that the medical tube 1 passes through a region between the first protrusion 21 of the first plate 20 and the second protrusion 31 of the second plate 30. Therefore, the spacing X between the first protrusion 21 and the second protrusion 31 is set to be larger than the diameter of the medical tube onto which the clamp is equipped.

The first plate 20 and the second plate 30, in particular, a structure related to a centering function of the second plate 30, will now be described in detail.

### [First Plate 20]

As described above, on the first plate 20, the first protrusion 21 which presses the medical tube 1 is formed. The first protrusion 21 is formed protruding toward the second plate 30 on an opposing surface 20S which opposes the second plate 30. In the present embodiment, the first protrusion 21 is formed over an entire width of the first plate 20 at a center part in the length direction of the first plate 20. In addition, a tip portion of the first protrusion 21 which contacts the medical tube 1 is formed in a rounded, semispherical shape in a side view of the clamp 10.

On an outer surface of the first plate 20 opposite from the opposing surface 20S, there is no large protrusion similar to the first protrusion 21. When the clamp 10 is locked, for example, the first plate 20 and the second plate 30 are pinched from above and below the structure, and pressure is applied to the second plate 30. Thus, a small unevenness 27 for preventing slippage is formed on the outer surface of the first plate 20. The thickness of the first plate 20 is, for example, about 0.5 mm ~ 3 mm in a flat portion where there is no large unevenness (this is similarly applicable to the second plate 30).

A pair of sidewalls 22 which extend in a standing manner are formed at respective ends in the width direction of the first plate 20. The pair of sidewalls 22 are formed to protrude toward the second plate 30 and to be higher than the first protrusion 21 on the opposing surface 20S of the first plate 20. Meanwhile, each of the sidewalls 22 is formed in a height and a width such that a length in the up-and-down direction (height) is shorter than a length of the locking portion 50 in the up-and-down direction, and the sidewall 22 does not contact the second plate 30 in the unlocked state and in the normal locked state.

The pair of sidewalls 22 is a structure related to the centering function of the second plate 30, and the second plate 30 is centered such that a centering guide 32 to be described below is positioned between the pair of sidewalls 22. The sidewalls 22 are desirably formed to be high in a range of not obstructing an appropriate locking manipulation, from the viewpoint of improving the centering function. In addition, the sidewalls 22 are formed in a thickness within a range of not obstructing an appropriate locking manipulation; that is, in a thickness to not interfere with the centering guide 32 when the pressure is applied on the second plate 30 straight toward the first plate 20.

The pair of sidewalls 22 are identical in shape and size, and are formed between a tip of the first plate 20 and the first protrusion 21, aligned in the width direction of the first plate 20. In the present embodiment, each of the sidewalls 22 is formed at a side nearer to the tip of the clamp 10 than the second protrusion 31 of the second plate 30, and an upper end of each of the sidewalls 22 is positioned at an upper position than a lower end of the second protrusion 31 in the unlocked state. An upper end surface of the sidewall 22 is formed, for example, parallel to the opposing surface 20S of the first plate 20. The sidewall 22 also functions as a tube guide for preventing squeezing-out of the medical tube 1 to the lateral direction from between the two plates.

### [Second Plate 30]

As described above, on the second plate 30, the second protrusion 31 which presses the medical tube 1 is formed. The second protrusion 31 is formed protruding toward the first plate 20 on an opposing surface 30S which opposes the first plate 20. In the present embodiment, the second protrusion 31 is formed over the entire width of the second plate 30 at a center part in the length direction of the second plate 30. In addition, a tip end portion of the protrusion 31 which contacts the medical tube 1 is more sharply pointed than the first protrusion 21, and the protrusion 31 is formed in an approximate V shape in side view. In this case, the force necessary for pressing the medical tube 1 is reduced, the maneuverability is improved, and the occlusion property of the medical tube 1 is improved. Such a shape particularly desirably acts on a medical tube having a small outer size. A lower end of the second protrusion 31 is desirably chamfered so as to not damage the medical tube. Alternatively, the tip end portion of the first protrusion 21 may be more sharply pointed than the second protrusion 31.

The lower end of the second protrusion 31 is positioned at a side nearer to the tip of the clamp 10 than the upper end of the first protrusion 21. With the placement of the protrusions in a shifted manner in the vertical direction such that the first protrusion 21 and the second protrusion 31 are not aligned in the up-and-down direction, even when the medical tube 1 is pressed by the sharply pointed second protrusion 31, the medical tube 1 tends not to be damaged, and the occlusion property of the medical tube 1 can be improved.

A pair of tube guides 36 protruding from respective ends in the width direction of the opposing surface 30S toward the first plate 20 are formed between the base end of the second plate 30 and the second protrusion 31. The pair of tube guides 36 prevent squeezing-out of the medical tube 1 in the lateral direction from between the two plates. In addition, similar to the first plate 20, a small unevenness 37 for preventing slippage is formed on an outer surface of the second plate 30.

On the opposing surface 30S of the second plate 30, a sloped surface which is inclined to be closer to the first plate 20 from the ends in the width direction toward the center in the width direction is formed. That is, the sloped surface is inclined with respect to the width direction in such a manner that the ends in the width direction of the second plate 30 are upper ends, and the center in the width direction is the lower end.

The sloped surface is formed to contact the sidewall 22 of the first plate 20 when an inappropriate locking manipulation is performed such as pushing the second plate 30 in a slanted direction. As will be described below in detail, with the sloped surface contacting the sidewall 22, the second plate 30 slides toward an inner side of the clamp 10, and the centering of the second plate 30 is performed. The sloped surface is desirably formed to be symmetric in the left and right with respect to the center in the width direction of the second plate 30. In this case, the same centering process is applied in cases where the second plate 30 is pushed in a slanted direction to the left and to the right.

The sloped surface of the present embodiment includes a first sloped surface 33 and a second sloped surface 34 having a smaller inclination angle θ than the first sloped surface 33, and the centering guide 32 is formed from the two sloped surfaces. The centering guide 32 (sloped surface) is formed between the tip of the second plate 30 and the second protrusion 31. Alternatively, the centering guide may be formed from one sloped surface (for example, only the first sloped surface 33), or may be formed from three or more sloped surfaces having different inclination angles θ from each other.

The centering guide 32 is formed in a slanted direction with respect to the opposing surface 30S from a region near the tip of the second protrusion 31 protruding toward the first plate 20 to a region near the tip of the second plate 30. In the present embodiment, in the side view of the clamp 10, the opposing surface 30S, the second protrusion 31, and the centering guide 32 are placed to respectively form one side of a triangle. In a region surrounded by the opposing surface 30, the second protrusion 31, and the centering guide 32, a reinforcement rib 38 having an approximate triangular shape in side view is formed. The reinforcement rib 38 is formed to be higher toward the second protrusion 31 in the center part in the width direction of the second plate 30.

According to the invention, a biting-prevention piece 35 protruding toward the first plate 20 is formed on the opposing surface 30S of the second plate 30. The biting-prevention piece 35 extends beyond a tip end position of the locking portion 50 toward the first plate 20. Because the opening 11 is formed between the second plate 30 and the locking portion 50 of the clamp 10, for example, a situation may be considered in which, during transport or storage, another clamp 10 enters the inside of the clamp 10, and the clamps cannot be easily separated. With the provision of the biting-prevention piece 35, such a disadvantage can be avoided.

The biting-prevention piece 35 is formed along the length direction from the tip of the second plate 30 to the second protrusion 31 at the center part in the width direction of the opposing surface 30S. With a connecting portion with the biting-prevention piece 35 on the second sloped surface 34 of the centering guide 32 being a lower end, the second sloped surface 34 is divided by the biting-prevention piece 35 into a one-end side and the other-end side in the width direction of the second plate 30. On the other hand, the lower end of the first sloped surface 33 formed at the center part in the width direction of the opposing surface 30S protrudes beyond a lower end of the biting-prevention piece 35 toward the first plate 20, and is thus not divided by the biting-prevention piece 35. Alternatively, the biting-prevention piece 35 may be omitted.

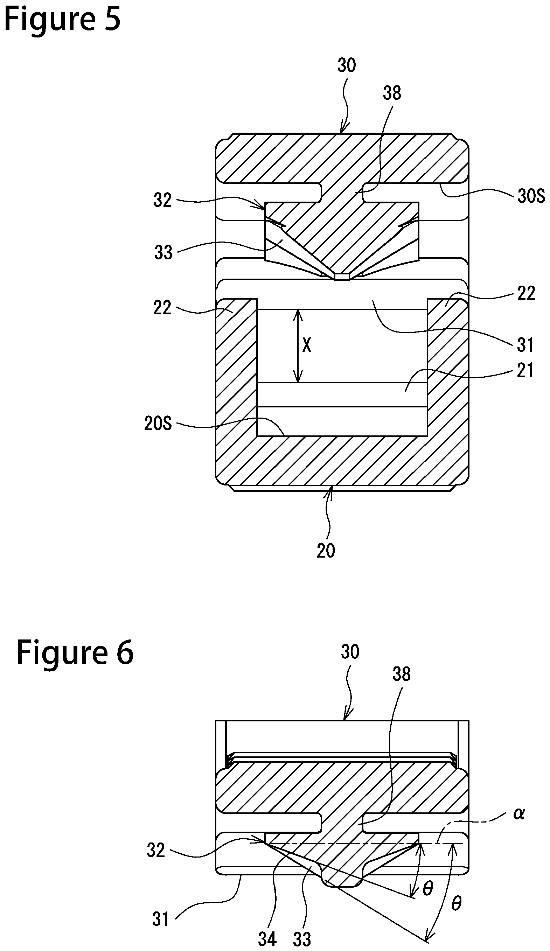

A structure of the centering guide 32 will now be described in detail with reference to FIGs. 5 and 6. FIG. 5 is a cross-sectional diagram along a line AA in FIG. 2, and FIG. 6 is a cross-sectional diagram along a line BB in FIG. 2.

As shown in FIG. 5, the centering guide 32 is formed to be positioned at the inside of the pair of sidewalls 22 formed on the first plate 20 in the normal locked state. That is, on the opposing surface 30S of the second plate 30, the centering guide 32 is formed in a region not overlapping the pair of sidewalls 22 in the up-and-down direction. With this configuration, when an appropriate locking manipulation is performed to push the second plate 30 straight, interference between the sidewall 22 and the centering guide 32 is prevented.

As shown in FIGs. 5 and 6, the sloped surface (the first sloped surface 33 and the second sloped surface 34) of the centering guide 32 is inclined by a predetermined angle θ with respect to the width direction of the second plate 30. A pair of the first sloped surfaces 33 are provided, forming an approximate V-shaped portion. The first sloped surfaces 33 are formed in such a manner that a vertex of the approximate V-shaped portion is positioned at an upper position in relation to the second protrusion 31, so as to reduce influences of the approximate V-shaped portion on the tube during the clamping. In addition, the sloped surfaces are formed in such a manner that the sloped surfaces can contact the pair of the sidewalls 22 when the spacing X between the first protrusion 21 and the second protrusion 31 is reduced by 10% ~ 90%. As described above, when the appropriate locking manipulation is performed, the sloped surface does not contact the sidewalls 22. In other words, the sloped surfaces are formed in such a manner that the sloped surfaces contact the sidewalls 22 when the second plate 30 is pushed in the direction in which the plates move toward each other in a state in which the second plate 30 is deviated in the lateral direction with respect to the first plate, and the spacing X is reduced by 10% ~ 90%.

Respective ends in the width direction of the first sloped surface 33 and the second sloped surface 34 are desirably placed near a position immediately above the sidewalls 22 in a range not obstructing the appropriate locking manipulation, and, in some configurations, may be formed immediately above the sidewalls 22. In the present embodiment, positions of the respective ends in the width direction of the first sloped surface 33 and the second sloped surface 34 coincide with each other, and the sloped surfaces are formed at a side slightly nearer to the center in the width direction of the second plate 30 than the positions immediately above the sidewalls 22.

The inclination angle θ of the sloped surface of the centering guide 32 with respect to the width direction of the second plate 30 is 10° ~ 60°. The inclination angle θ is an angle of the sloped surface with respect to a virtual line α which is parallel to the width direction of the second plate 30 in a cross section (cross section shown in FIG. 6) in which the second plate 30 is cut in the width direction, perpendicular to the lower end of the sloped surface. When, as in the present embodiment, the sloped surface includes the first sloped surface 33 and the second sloped surface 34, at least the first sloped surface 33 having a steeper inclination is formed with the angle θ of 10° ~ 60°, and, desirably, both sloped surfaces are formed with the angle θ of 10° ~ 60°.

As the angle θ becomes larger, a component force in the lateral direction to the sidewall 22 in the slanted direction becomes stronger, and the centering can more reliably and easily performed against the frictional force. However, when the angle θ is too large, the slope height becomes high, resulting in firmer contact with the tube in the unlocked state, a higher frictional force, and, consequently, difficulty in the centering process. On the other hand, when the inclination angle θ of the sloped surface is less than 10°, it becomes more difficult for the force, for sliding the second plate 30 toward the center in the width direction of the first plate 20 when the sloped surface contacts the sidewall 22, to act, and, as a consequence, the centering process of the second plate 30 cannot be performed to a sufficient degree.

In the centering guide 32, the inclination angle θ of at least the first sloped surface 33 (hereinafter referred to as an "inclination angle θ1") is desirably 25° or greater, and is more desirably 30° or greater, from the viewpoint of improving the centering function. An upper limit of the inclination angle θ1 is 45°, or less than or equal to 40°, from the viewpoint of a molding characteristic of the clamp 10, prevention of interference with the medical tube 1, or the like. Examples of a desirable range of the inclination angle θ1 include 25° ~ 45°, 30° ~ 45°, and 30° ~ 40°. Desirably, an inclined surface having the inclination angle θ1 in the above-described range is formed at least partially on the first sloped surface 33, and, in this case, the centering process of the second plate 30 can be made smoother.

On the other hand, the inclination angle θ of the second sloped surface 34 (hereinafter, referred to as an "inclination angle θ2") is smaller than the inclination angle θ1 of the first sloped surface 33, and may be, for example, 10° or greater and less than 25°, or 20° or greater and less than 25°. When the centering guide is formed from one sloped surface, the inclination angle θ of the sloped surface is desirably 25° or greater, and is more desirably 30° or greater, similar to the first sloped surface 33.

In the present embodiment, the first sloped surface 33 and the second sloped surface 34 are both flat surfaces without unevenness. In addition, the second sloped surface 34 is formed at a side nearer to the tip of the second plate 30 than the first sloped surface 33. When two sloped surfaces having different inclination angles θ are formed as in the present embodiment, the sloped surface with the smaller inclination angle θ is formed at a side nearer to the tip of the first plate 20 than the sloped surface with the larger inclination angle θ. It is sufficient that the first sloped surface 33 can contact a portion of the sidewall 22, and the surface is formed to be longer in the lateral direction than in the vertical direction. With such a configuration, sink marks which are formed during the molding due to an increase in the thickness of the second plate 30 can be reduced, along with the adverse influences of the sloped surface on the tube during the clamping.

The centering guide 32 is formed at a position to contact the sidewall 22 when the second plate 30 is pushed in a slanted direction and the spacing X is reduced by 10% ~ 90%, desirably, 30% ~ 70%. If the centering guide 32 contacts the sidewall 22 at a stage of a small reduction percentage of the spacing X, the centering process would be performed when the frictional force is relatively small. Thus, with the above-described structure, a situation in which the centering does not function due to the frictional force can be reduced. A length in the vertical direction of the first sloped surface 33 is, for example, 10% ~ 50% of the length in the vertical direction of the sidewall 22. As the length in the vertical direction of the first sloped surface 33 becomes shorter, the contact area with the sidewall 22 becomes smaller, resulting in a smaller frictional resistance during the centering, and ease of manipulation. However, when the length in the vertical direction is too short, there is a possibility that the sloped surface does not contact the sidewall 22 when the connecting portion 40 is significantly distorted. Therefore, an appropriate relationship is desirable.

The centering guide 32 is desirably formed in such a manner that the centering guide 32 contacts the sidewall 22 before the second plate 30 is pushed in the slanted direction and the spacing X is reduced by 50%. In this case, the centering of the second plate 30 can be realized quickly, and the maneuverability is further improved. In the side view of the clamp 10 in the unlocked state, the spacing between the lower end of the first sloped surface 33 and the upper end of the sidewall 22 is, for example, 10% ~ 80%, and is desirably 20 % ~ 50%, of the spacing X between the first protrusion 21 and the second protrusion 31.

Because the first sloped surface 33 protrudes more toward the first plate 20 than the second sloped surface 34 does, when the inappropriate locking manipulation is performed such as pushing the second plate 30 in the slanted direction, the first sloped surface 33 first contacts the sidewall 22. On the other hand, when strong pressure is applied to the second plate 30 toward the connecting portion 40, the second sloped surface 34 which is formed on the side nearer to the tip of the second plate 30 than the first sloped surface 33 contacts the sidewall 22, and, thus, the second plate 30 is centered using the second sloped surface 34.

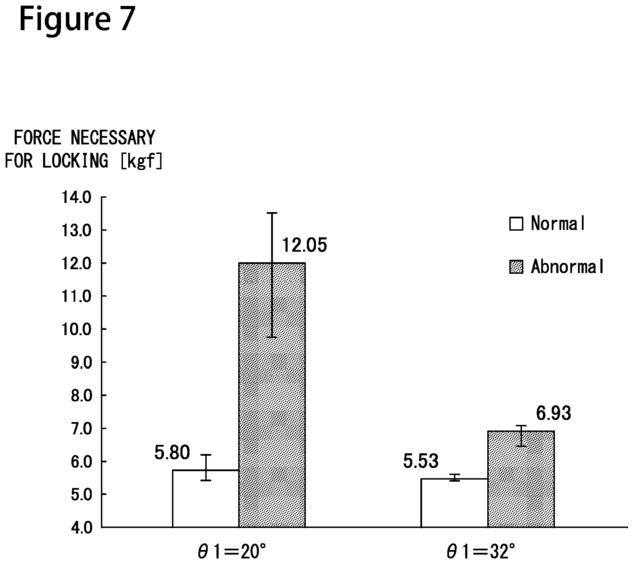

FIG. 7 is a diagram showing a relationship between the inclination angle θ1 of the first sloped surface 33 and the force necessary for locking the clamp 10 (pressurization force of the second plate 30). The pressurization force was measured using a precision universal testing machine autograph ("AG Xplus" manufactured by Shimadzu Corporation). In this experiment, for the inclination angles θ1 of 20° and 32°, pressurization forces were measured 5 times for each inclination angle, and average values were determined. For the clamp having θ1 of 20°, the shape of the tip portion of the second protrusion 31 which contacts the medical tube 1 was set to a semispherical shape in side view, similar to the first protrusion 21. For the medical tube 1, a flexible tube having a diameter similar to the spacing X was used.

The configuration labeled "Normal" in FIG. 7 indicates a case where the clamp is locked by an appropriate manipulation to push the second plate 30 straight toward the first plate 20. The configuration labeled "Abnormal" in FIG. 7 indicates a case where the second plate 30 is pushed in the slanted direction so that a region of the first sloped surface 33 near the lower end contacts the sidewall 22, and the clamp is locked after the centering of the second plate 30.

As shown in FIG. 7, when the appropriate locking manipulation is performed, because the first sloped surface 33 of the second plate 30 does not contact the sidewall 22 of the first plate 20, there is no significant difference in the pressurization force between different inclination angles θ1. In the clamp having θ of 32°, the pressurization force is smaller by about 5% than the clamp having θ1 of 20°, but this difference may be considered to be caused by the shape of the tip end portion of the second protrusion 31. In other words, by forming the tip end portion of the second protrusion 31 in the approximate V shape in side view, it becomes possible to reduce the force necessary for locking, and to improve maneuverability.

On the other hand, when the inappropriate locking manipulation in which the second plate 30 is pushed in the slanted direction is performed, there is a significant difference in the pressurization force depending on the inclination angle θ1. As shown in FIG. 7, in the clamp having θ of 32°, the pressurization force is significantly reduced in comparison to the clamp with θ1 of 20°. In other words, in the clamp having θ of 32°, in comparison to the clamp having θ1 of 20°, when the inappropriate manipulation is performed, it is easier to center the second plate 30 and to secure the normal locked state.

When the inclination angle θ1 of the first sloped surface 33 is less than 10°, the pressurization force necessary for locking when the inappropriate locking manipulation is performed is significantly increased, and the centering of the second plate 30 is difficult. When the inclination angle θ1 is 10° or greater, the second plate 30° can be centered, and, when the inclination angle θ1 is 25° or greater, in particular, 30° or greater, the pressurization force necessary for the locking is specifically reduced, and the maneuverability is significantly improved.

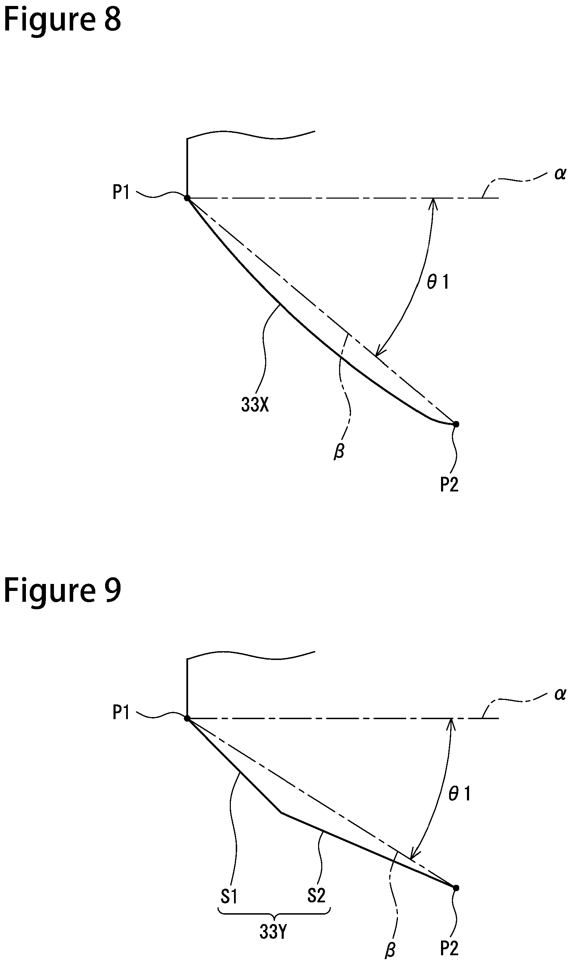

FIGs. 8 and 9 are diagrams showing alternative configurations of the first sloped surface 33 of the centering guide 32. A first sloped surface 33X exemplified in FIG. 8 is formed to be curved in a manner to be convex down, so that a distance between the sloped surface and the sidewall is reduced, and the centering is performed at an earlier stage when the plate is pushed in the slanted direction. A first sloped surface 33Y exemplified in FIG. 9 differs from the first sloped surface 33 formed in a flat shape in that the sloped surface 33Y is curved in a manner to be convex down, and there is a point of change of an angle. With such a configuration, the component force in the width direction when the sloped surface contacts the sidewall can be increased and the length in the height direction of the sloped surface can be reduced. Each of the first sloped surfaces 33X and 33Y is a convex surface protruding downward in relation to a virtual line β connecting an upper end P1 and a lower end P2 of the sloped surface in the cross section in which the second plate 30 is cut in the width direction, perpendicular to the lower end of the sloped surface. In this case, an angle between the virtual line α parallel to the width direction of the second plate 30 and the virtual line β is assumed to be the inclination angle θ1.

Alternatively, the sloped surface may be a curved or bent concave surface which is convex up. In this case also, the angle between the virtual line α and a virtual line β connecting the upper end P1 and the lower end P2 of the sloped surface is assumed to be the inclination angle θ. The sloped surface may be a concave surface, but desirably, the sloped surface is a flat surface or a convex surface. The first sloped surface 33Y exemplified in FIG. 9 is formed from two flat surfaces S1 and S2 having different inclination angles with respect to the virtual line α, and desirably, both of the flat surfaces S1 and S2 are inclined by an angle of 25° or greater with respect to the virtual line α.

As described, with the clamp 10 having the above-described structure, even when inappropriate locking manipulation is performed, the second plate 30 can be centered at the normal position where the medical tube 1 can be reliably occluded, and the normal locked state can be easily secured. Therefore, with the clamp 10, the medical tube 1 can be quickly and reliably occluded. In addition, even when the clamp 10 is equipped on a medical tube 1 having a large diameter, the centering of the second plate 30 can be easily performed.

In particular, by providing the centering guide 32 including the sloped surface having the inclination angle θ with respect to the width direction of the second plate 30 of 25° ~ 45°, and more desirably, 30° ~ 45°, the force necessary for the centering of the second plate 30 is significantly reduced, and a smoother centering is enabled.

The embodiment described above can be suitably changed in design within a range not adversely affecting the objective of the present invention. For example, in the embodiment described above, a clamp 10 formed from a resin molded structure which is integrally molded is exemplified, but alternatively, the clamp may be produced by assembling a plurality of components. In addition, in the embodiment described above, the sidewall 22 is formed on the first plate 20 and the sloped surface (the first sloped surface 33 and the second sloped surface 34) is formed on the second plate 30, but alternatively, the sloped surface may be formed on the first plate and the sidewall may be formed on the second plate.

### REFERENCE SIGNS LIST

1 MEDICAL TUBE; 10 MEDICAL TUBE CLAMP (CLAMP); 11 OPENING; 20 FIRST PLATE; 21 FIRST PROTRUSION; 22 SIDEWALL; 27, 37 UNEVENNESS; 30 SECOND PLATE; 31 SECOND PROTRUSION; 32 CENTERING GUIDE; 33 FIRST SLOPED SURFACE; 34 SECOND SLOPED SURFACE; 35 BITING-PREVENTION PIECE; 36 TUBE GUIDE; 38 REINFORCEMENT RIB; 40 CONNECTING PORTION; 41, 51 TUBE PASS-THROUGH HOLE; 50 LOCKING PORTION; 52 HOOK.

## Claims

1. A medical tube clamp (10) which presses and occludes a flexible medical tube (1), the medical tube clamp (10) comprising:
a first plate (20) on which a first protrusion (21) is formed;
a second plate (30) placed opposing the first plate (20), and on which a second protrusion (31) which presses the medical tube (1) along with the first protrusion (21) is formed;
a connecting portion (40) that connects one end of the first plate (20) in a length direction and one end of the second plate (30) in a length direction, and that is elastically deformable so as to allow the first plate (20) and the second plate (30) to be closer to each other; and
a locking unit (50) that is formed extending from the first plate (20) toward the second plate (30), that locks an other end of the second plate (30) in the length direction, and that maintains an occluded state of the medical tube (1), wherein
a pair of sidewalls (22) which extend in a standing manner are formed on both ends in a width direction on the first plate (20),
a biting-prevention piece (35) is formed extending from the other end of the second plate in the length direction (30) to the second protrusion (31) along the length direction on a center in a width direction of the second plate (30), the biting-prevention piece (35) protruding toward the first plate (20),
a sloped surface (33) is formed on the second plate (30), the sloped surface (33) being inclined toward the first plate (20) from both ends in the width direction toward the center in the width direction of the second plate (30), and
an inclination angle θ of the sloped surface (33) with respect to the width direction of the second plate (30) is 10° ~ 60°, and **characterized in that** the sloped surface (33) protrudes beyond the biting-prevention piece (35) toward the first plate (20) at the center in the width direction of the second plate (30).

2. The medical tube clamp (1) according to claim 1, wherein
the inclination angle θ of the sloped surface (33) is 25° ~ 45°.

3. The medical tube clamp (1) according to claim 1 or 2, wherein
one of the first protrusion (21) and the second protrusion (31) is more sharply pointed than the other of the first protrusion (21) and the second protrusion (31).

## Patentansprüche

1. Medizinische Schlauchklemme (10), die einen flexiblen medizinischen Schlauch (1) zusammendrückt und verschließt, wobei die medizinische Schlauchklemme (10) umfasst:
eine erste Platte (20), auf der ein erster Vorsprung (21) ausgebildet ist;
eine zweite Platte (30), die gegenüber der ersten Platte (20) angeordnet ist und auf der ein zweiter Vorsprung (31) ausgebildet ist, der den medizinischen Schlauch (1) zusammen mit dem ersten Vorsprung (21) zusammendrückt;
einen Verbindungsabschnitt (40), der ein Ende der ersten Platte (20) in einer Längsrichtung und ein Ende der zweiten Platte (30) in einer Längsrichtung verbindet, und der elastisch verformbar ist, um zu ermöglichen, dass die erste Platte (20) und die zweite Platte (30) näher aneinander sind; und
eine Verriegelungseinheit (50), die von der ersten Platte (20) zu der zweiten Platte (30) sich erstreckend ausgebildet ist, die ein anderes Ende der zweiten Platte (30) in der Längsrichtung verriegelt, und die einen verschlossenen Zustand des medizinischen Schlauchs (1) aufrechterhält, wobei
ein Paar stehend verlaufende Seitenwände (22) an beiden Enden in einer Breitenrichtung an der ersten Platte (20) ausgebildet sind,
ein Beißschutzteil (35) ausgebildet ist, das sich vom anderen Ende der zweiten Platte in Längsrichtung (30) zu dem zweiten Vorsprung (31) entlang der Längsrichtung in einer Mitte in einer Breitenrichtung der zweiten Platte (30) erstreckt, wobei das Beißschutzteil (35) in Richtung der ersten Platte (20) vorsteht,
eine schräge Fläche (33) an der zweiten Platte (30) ausgebildet ist, wobei die schräge Fläche (33) in Richtung der ersten Platte (20) von beiden Enden in der Breitenrichtung zur Mitte in der Breitenrichtung der zweiten Platte (30) geneigt ist, und
ein Neigungswinkel θ der schrägen Fläche (33) in Bezug auf die Breitenrichtung der zweiten Platte (30) 10° - 60° beträgt, und **dadurch gekennzeichnet, dass**
die schräge Fläche (33) in der Mitte in Breitenrichtung der zweiten Platte (30) über das Beißschutzteil (35) in Richtung der ersten Platte (20) vorsteht.

2. Medizinische Schlauchklemme (1) nach Anspruch 1, wobei
der Neigungswinkel θ der schrägen Fläche (33) 25° ∼ 45° beträgt.

3. Medizinische Schlauchklemme (1) nach Anspruch 1 oder 2, wobei
einer des ersten Vorsprungs (21) und des zweiten Vorsprungs (31) spitzer ist als der andere des ersten Vorsprungs (21) und des zweiten Vorsprungs (31).

## Revendications

1. Pince à tube médical (10) qui comprime et bouche un tube médical flexible (1), la pince à tube médical (10) comprenant :
une première plaque (20) sur laquelle est formée une première saillie (21) ;
une seconde plaque (30) placée à l'opposé de la première plaque (20) et sur laquelle est formée une seconde saillie (31) qui comprime le tube médical (1) conjointement avec la première saillie (21) ;
une partie de raccordement (40) qui raccorde une extrémité de la première plaque (20) dans une direction de longueur et une extrémité de la seconde plaque (30) dans une direction de longueur, et qui est élastiquement déformable, afin de permettre à la première plaque (20) et à la seconde plaque (30) d'être plus près l'une de l'autre ; et
une unité de verrouillage (50) qui est formée en s'étendant de la première plaque (20) vers la seconde plaque (30), qui verrouille une autre extrémité de la seconde plaque (30) dans la direction de longueur et qui maintient un état bouché du tube médical (1), dans laquelle :
une paire de parois latérales (22) qui s'étend à la verticale, est formée sur les deux extrémités dans une direction de largeur sur la première plaque (20),
une pièce de prévention de morsure (35) est formée en s'étendant de l'autre extrémité de la seconde plaque dans la direction de longueur (30) vers la seconde saillie (31) le long de la direction de longueur sur un centre dans une direction de largeur de la seconde plaque (30), la pièce de prévention de morsure (35) faisant saillie vers la première plaque (20),
une surface inclinée (33) est formée sur la seconde plaque (30), la surface inclinée (33) étant inclinée vers la première plaque (20) à partir des deux extrémités dans la direction de largeur vers le centre dans la direction de largeur de la seconde plaque (30), et
un angle d'inclinaison θ de la surface inclinée (33) par rapport à la direction de largeur de la seconde plaque (30) est de 10° ∼ 60°, et **caractérisée en ce que** :
la surface inclinée (33) fait saillie au-delà de la pièce de prévention de morsure (35) vers la première plaque (20) au centre dans la direction de largeur de la seconde plaque (30).

2. Pince à tube médical (1) selon la revendication 1, dans laquelle :
l'angle d'inclinaison θ de la surface inclinée (33) est de 25° ∼ 45°.

3. Pince à tube médical (1) selon la revendication 1 ou 2, dans laquelle :
l'une parmi la première saillie (21) et la seconde saillie (31) est nettement plus pointue que l'autre parmi la première saillie (21) et la seconde saillie (31).
